# EUROPEAN PATENT SPECIFICATION

(11) **EP 3 234 194 B1**
(45) Date of publication and mention of the grant of the patent: **09.09.2020**
(21) Application number: 15834679.1
(22) Date of filing: 18.12.2015
(51) Int. Cl.: G01N 33/573

(54) **NEW METHOD FOR DETECTING HUMAN BUTYRYLCHOLINESTERASE**
NEUES VERFAHREN ZUM NACHWEIS VON MENSCHLICHER BUTYRYLCHOLINESTERASE
NOUVEAU PROCÉDÉ DE DÉTECTION DE BUTYRYLCHOLINESTÉRASE HUMAINE

(30) Priority: 18.12.2014 EP 14198901
(43) Date of publication of application: 25.10.2017
(73) Proprietor: Nederlandse Organisatie voor toegepast- natuurwetenschappelijk onderzoek TNO, 2595 DA 's-Gravenhage (NL)
(72) Inventor: DE BOER, Bart Michiel, 2595 DA 's-Gravenhage (NL); LANGENBERG, Johannes Pieter, 2595 DA 's-Gravenhage (NL); BISCHOFF, Lucas Jacobus Marie, 2595 DA 's-Gravenhage (NL); VAN DER SCHANS, Marinus Johannes, 2595 DA 's-Gravenhage (NL); NOORT, Daniel, 2595 DA 's-Gravenhage (NL)
(74) Representative: V.O.
(86) International application number: PCT/NL2015/050883
(87) International publication number: WO 2016/099275

(56) References cited:
- WO-A2-2009/064321
- US-B1- 7 796 262
- DAN DU ET AL: "Magnetic Electrochemical Sensing Platform for Biomonitoring of Exposure to Organophosphorus Pesticides and Nerve Agents Based on Simultaneous Measurement of Total Enzyme Amount and Enzyme Activity", ANALYTICAL CHEMISTRY, vol. 83, no. 10, 15 May 2011 (2011-05-15), pages 3770-3777, XP055196577, ISSN: 0003-2700, DOI: 10.1021/ac200217d
- SUN ET AL: "Development of a MALDI-TOF-MS Method to Identify and Quantify Butyrylcholinesterase Inhibition Resulting from Exposure to Organophosphate and Carbamate Pesticides", JOURNAL OF THE AMERICAN SOCIETY FOR MASS SPECTROMETRY, ELSEVIER SCIENCE INC, US, vol. 18, no. 4, 24 March 2007 (2007-03-24) , pages 698-706, XP022000519, ISSN: 1044-0305, DOI: 10.1016/J.JASMS.2006.11.009
- Frank Vollmer ET AL: "Optical resonator based Biomolecular sensors and logic devices", Journal of the Indian Institute of Science, 1 April 2012 (2012-04-01), pages 233-252, XP055097212, Retrieved from the Internet: URL:http://www.mpl.mpg.de/research-groups/ bfp/publications/IISc_review_Vollmer.pdf
- MARTIN BAASKE ET AL: "Optical Resonator Biosensors: Molecular Diagnostic and Nanoparticle Detection on an Integrated Platform", CHEMPHYSCHEM, vol. 13, no. 2, 1 February 2012 (2012-02-01), pages 427-436, XP055097038, ISSN: 1439-4235, DOI: 10.1002/cphc.201100757
- JENNIFER L. S. SPORTY ET AL: "Immunomagnetic Separation and Quantification of Butyrylcholinesterase Nerve Agent Adducts in Human Serum", ANALYTICAL CHEMISTRY, vol. 82, no. 15, 1 August 2010 (2010-08-01), pages 6593-6600, XP055196581, ISSN: 0003-2700, DOI: 10.1021/ac101024z
- BAJGAR J ET AL: "Inhibition of blood cholinesterases following intoxication with VX and its derivatives", JOURNAL OF APPLIED TOXICOLOGY, WILEY HEYDEN LTD, GB, vol. 27, no. 5, 14 February 2007 (2007-02-14), pages 458-463, XP002574479, ISSN: 0260-437X, DOI: 10.1002/JAT.1226

## Description

The invention relates to the field of detection of biological compounds, particularly for the detection of butyrylcholinesterase. Such a detection may be advantageously used in the detection of the duration and severity of exposure to cholinesterase inhibiting nerve gases or pesticides.

### INTRODUCTION

Organophosphorous compounds are a group of the most toxic compounds that are known. Many of those have been used in warfare as nerve gas, such as sarin, tabun and VX. Organophosphorous toxins are also used as pesticides and insecticides.

Intoxication with these compounds brings a serious risk. The potential for civilian exposure to these compounds is greater today than at any time in history. Several events in recent history, such as the gassing of Kurds in Iraq in 1988, the attack in the subway in Tokyo in 1995 and the recent - still debated - gas attacks in Syria, indicate a demand for more accurate and sensitive methods to monitor exposure to cholinesterase inhibiting agents.

Currently one of the most reliable methods to measure exposure to organophosphorous compounds is by using inhibition of bytyrylcholinesterase (BuChE or BChE) as an indicator. Whereas originally such an assay was performed based on a mass spectrographic detection (Polhuijs,M. et al., 1997, Toxicol. Appl. Pharmacol. 146:156-161), more recently MALDI-TOF methods have been used (e.g. Doom, J.A. et al., 2001, Toxicol. Appl. Pharmacol. 176:73-80; Fidder, A. et al., 2002, Chem. Res. Toxicol. 15:582-590; Sun, J. and Lynn B.C., 2007, J. Am. Soc. Mass Spectrom. 18:698-706) and also other enzymatic assays have been reported (Du, D. et al., 2011, Anal. Chem. 83:3770-3777; Du, D. et al., 2012, Anal. Chem. 84:1380-1385).

Although the mass spectrometric assays have been found to perform very well in measuring exposure to organophosphorous compounds, the equipment needed is large and heavy and can therefore not being used in field situations. Further, the mass spectrometric assay requires a relatively long analysis time (typically about 4 hours), due to the sample work-up that is needed, as well as highly trained personnel. Therefore, there is a need for a fast, reliable and low weight device for performing the assay in the field.

### SUMMARY OF THE INVENTION

The inventors now have developed an optical resonator biosensor for the detection of butyrylcholinesterase comprising two optical resonator biosensor systems in which in the first optical resonator biosensor system a probe is attached to the resonator wherein said probe is able to bind butyrylcholinesterase, preferably wherein said probe is able to bind uninhibited butyrylcholinesterase. In a further preferred embodiment the optical resonator biosensor provides in the second optical resonator biosensor system an antibody that is attached to the resonator, wherein said antibody is able to bind butyrylcholinesterase, preferably wherein said antibody is able to bind both inhibited and uninhibited butyrylcholinesterase.

In a further preferred embodiment the optical resonator biosensor is provide with a sample, applied in such a way that it comes into contact with both optical resonator biosensor systems., preferably by applying the the sample via a capillary track. In a further preferred embodiment, both optical resonator biosensor systems are placed along one capillary.In such a system preferably the amount of probes in said first optical resonator biosensor is sufficient large to capture all the uninhibited butyrylcholinesterase. Alternatively, also preferred is a biosensor wherein one optical resonator biosensor system is placed along one branch of the capillary, while the other optical resonator biosensor system is placed along an other branch of the capillary.

A further preferred embodiment is a biosensor wherein the resonator is a ring resonator.

Another further preferred embodiment is a biosensor wherein the resonator biosensor is a waveguide, preferably wherein said waveguide is part of an interferometer.

A further part of the invention is a method for determining the exposure to an organophosphorous compound by measuring the amounts of inhibited, uninhibited and total butyrylcholinesterase in a sample by assaying said sample with an optical resonator biosensor according to the invention.

Also part of the invention is the use of an optical resonator biosensor for the determination of the exposure to an organophosphorous compound, wherein said optical resonator biosensor is an optical resonator biosensor according to the invention.

In the above mentioned optical resonator biosensor, method and use the butyrylcholinesterase is a human butyrylcholinesterase. Further, in the above mentioned optical resonator biosensor, method and use the probe comprises the following compound: 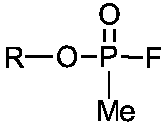 in which R is an spacer chain with more than 10 C-atoms, which is able to be bound to the sensor surface. Preferably said spacer chain is a substituted or unsubstituted, linear or branched hydrocarbon chain of more than 10 carbon atoms, wherein one to three of the C atoms may be replaced by a heteroatom selected from the group of O, N and S, and wherein the chain may have substitutions selected from the group of halogen, phenyl, linear or branched O-C₁-C₆ alkyl, linear or branched alkoxy, nitro, cyano or methylsulfinyl. Examples of such probes are: 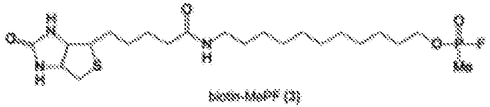 and

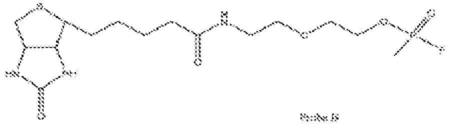

In a further preferred embodiment the antibody that binds to butyrylcholinesterase in the above mentioned optical resonator biosensor, method and use is monoclonal antibody 3E8.

### DESCRIPTION OF THE FIGURE

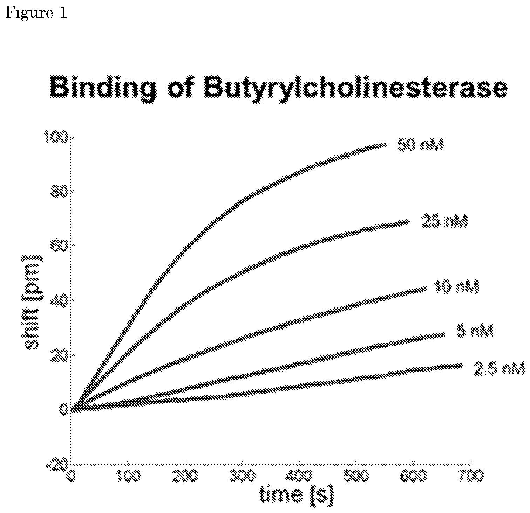

Fig. 1 shows the dose-dependent shift of resonsnace wavelength from various doses of human butyrylcholinesterase applied to a biosensor on a ring resonator. The Y-axis denots the shift in wavelength, while the x-axis shows the time in seconds.

### DETAILED DESCRIPTION

Butyrylcholinesterase is an enzyme that is inhibited when an organosphosphorous compound, such as a nerve gas or a pesticide, binds to its active site. Inhibition of BuChE in itself does not give any toxic effects (in comparison to inhibition of acetylcholinesterase, AChE, which causes the toxicity demonstrated by the organophosphorous compounds), but it can ideally be used as a biomarker. The concentration of inhibited BuChE in comparison with the total BuChE concentration conveys information whether or not a subject has been exposed to an organophosphorous compound, as well as an indication of the extent of the exposure.

Most systems use detection of inhibited BuChE and indeed this indicates exposure more qualitatively. For a quantitative assay, however, preferably also detection of total BuChE is achieved with immunological methods.

To detect total BuChE preferably an antibody that is able to bind to the enzyme is used. To detect the amount of inhibited BuChE it is easier to achieve this by detecting the amount of uninhibited BuChE, which can be done by detection of binding to a probe that is able to bind uninhibited BuChE. Of course then the amount of inhibited BuChE is the total amount of BuChE minus the amount of uninhibited BuChE. As used in the present application, the word "probe" is used for a molecule that is able to bind uninhibited BuChE.

As used in the present application, the term 'inhibited BuChE' means an enzyme that is inactivated by binding to a target molecule, said target molecule being an organophosporous compound as provided by a nerve gas or pesticide, or, alternatively, provided as probe in the assay of the present invention. Accordingly, an 'uninhibited BuChE' of the present invention is a molecule wherein the recognition site for an organophosphorous compound has not been occupied, which in general means that the enzyme is still able to exert its enzymatic action. Further, the 'inhibited' or 'uninhibited' state is not altered by binding to the ant-BuChE antibody as defined herein.

As used herein an anti-BuChE antibody, often being referred to as 'the antibody' in the present description, is preferably a monoclonal antibody that is able to bind to both inhibited and uninhibited BuChE. Such antibodies are known in the art (e.g. Wang, L. et al., 2011, Analytica Chimica Acta, 693(5):1-6; Sproty, J.L., et al. 2010, Anal. Chem. 82:6593-6600). One example is monoclonal antibody 3E8. An alternative antibody might be the goat polyclonal IgG N-15 Sc-46803 (against N-terminal part of human BuChE; Santa Cruz Biotechnology).

In the present invention the detection of the total and uninhibited BuChE is achieved by using an optical detection method, an optical resonator biosensor or ring resonator..An optical resonator biosensor works through monitoring resonance wavelength shifts which occur in optical response of the ring resonator, i.e. its transmission as function of wavelength. The binding of the biomolecule to another molecule (a probe, or an antibody) or of a biomolecule-antibody or biomolecule-probe complex to the sensor will result in a shift of resonance wavelengths allowing detection of bound vs unbound biomolecule.

An optical resonator biosensor consists of three parts: 1) a biorecognition element, which in the present invention is a probe or an antibody that is able to bind BuChE, more preferably human BuChE; 2) an optical transducer (the optical resonator) and 3) an electronic readout scheme for measuring and recording optical frequency shifts (Vollmer, F. and Baaske, M. 2012, ChemPhysChem 13:427-436). The optical resonator may be in the form of a glass microsphere, but may also be formed by micro-capillaries and liquid-core optical ring resonators (LCORR), micro-toroids, micro-disks, photonic crystal cavities and micro-rings. Preferably in the present invention a ring resonator is used. The general lay-out of such micro-rings is known in the art (e.g. Lin, S.Y. et al., 2010, Nano Lett. 10:2408-2411; Nitkowski A. et al., 2011, Biomed. Optics Express 2:271-277; Carlborg, C.F. et al., 2010, Lab on a Chip 10:281-290).

The advantages of the optical resonator biosensor are that a complete test system can be obtained on a very small scale: the actual measuring device can be provided as a lab-on-a-chip technology, where e.g. a ring resonator maybe used with a diameter of 5- 100 pm. This allows testing in the field.

Further, the speed of detection can be very high (within a few seconds to a few minutes), while the amount of material needed, the sample volume, may be minimal (in the nanoliter or microliter range). On top of all of these benefits, the optical resonator biosensor systems usually combine a very high sensitivity with a good specificity.

The function of a ring resonator is characterized by a transmission as function of wavelength that is dependent on the ambient refractive index of the ring. This response shows periodic resonances. By monitoring the resonances wavelength shifts as function of the time, refractive index variations can accurately and immediately be measured. As such, the binding of molecules to its receptors (such as binding of the probe or antibody to the enzyme) will result in a shift of resonance wavelengths, the amount of shift corresponding to the amount of molecules bound.

A sample may be obtained from any body fluid that contains the enzyme butyrylcholinesterase, but preferably a blood sample. Since only a small amount of sample is needed to be applied to the testing device, blood from a finger prick is an ideal source, since it is readily obtained even under field conditions. However, also blood samples from other sources (e.g. venipuncture, or bleeding wounds) may be used.

The optical resonator biosensor in the present invention at least needs to measure the uninhibited BuChE level. For this purpose the resonator is provided with a probe that is able to bind uninhibited BuChe, more preferably uninhibited human BuChE (HuBuChE). This probe advantageously is a compound that is able to bind to the BuChe at the active site, i.e. a compound that resembles an organophosphorous compound as defined above or any other molecule that is able to bind to the active site of the enzyme. Preferably, this is a compound with the general formula: 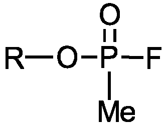 in which R is an spacer chain with more than 10 C-atoms, which is able to be bound to the sensor surface. Preferably said spacer chain is a substituted or unsubstituted, linear or branched hydrocarbon chain of more than 10 carbon atoms, wherein one to three of the C atoms may be replaced by a heteroatom selected from the group of O, N and S, and wherein the chain may have substitutions selected from the group of halogen, phenyl, linear or branched O-C₁-C₆ alkyl, linear or branched alkoxy, nitro, cyano or methylsulfinyl. Alternatively, the chain may comprise a (poly)ethylene glycol chain. Preferably the part of R that is capable of binding to the sensor is a biotin moiety or an amino moiety. In the fall of a biotin moiety, the sensor binds with an avidin moiety at the surface of the sensor. In the fall of an amino moiety, said moiety binds with a reactive carboxyl-group that is present at the surface of the biosensor. The skilled person will know how to provide the biosensor surface with the above-mentioned components for binding with their counterparts on the probe. Examples of suchprobes are:: 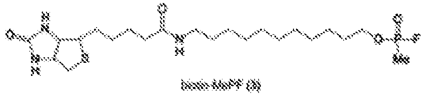 and

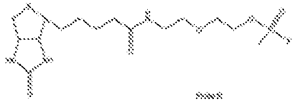

It will be clear to the skilled person that the R group may vary considerably and primarily acts as a spacer to prevent interaction of the bound enzyme with the biosensor.

Next to the embodiments where the probe is bound to the surface of the sensor, it is also possible that an aqueous composition comprising free probe is added to the sample on the sensor. In such a case, the probe may first bind with the enzyme and then together with the enzyme may be bound to the sensor surface. Whether or not the actual binding to the enzyme in such a situation takes place before or after the probe has attached itself to the sensor surface is not relevant.

When such a molecule is immobilized on a refractive index sensor, such as a ring resonator, the binding of the molecule by BuChE or HuBuChE or the binding of the enzyme-antibody or enzyme-probe complex, can be detected as refractive index variations by monitoring the shift of the resonance wavelength as a function of time. Since the amount of shift corresponds to the amount of molecules bound, this provides a reliable, quick and a quantitative detection of the amount of uninhibited BuChE.

In one embodiment of the present invention, a test system comprises at least two sensors, one for measuring the amount of uninhibited BuChE, as indicated above, and one that measures the amount of inhibited BuChE. This latter measurement is achieved by measuring the total amount of BuChE, from which the amount of uninhibited BuChE then again should be subtracted (inhibited BuChE + uninhibited BuChE = total BuChE). As indicated above, this is achieved by measuring the binding of BuChE to an antibody, wherein said antibody is capable of binding both inhibited and uninhibited BuChE. The antibody may be bound to the surface of the sensor or may first react freely in the test (sample) solution with the BuChE and after reaction as antibody-enzyme complex be bound to the sensor. The skilled person is capable of finding ways how to bind the antibody to the sensor (e.g. by immobilising on the sensor an antibody that recognizes the anti-BuChe-antibody). An example of an antibody that is able to bind to both inhibited BuChE and uninhibited BuChE may be the monoclonal antibody 3E8, which is commercially available (e.g. from Thermo Fisher Scientific, Inc, Rockford, Illinois, USA). Also other commercially available antibodies which are specific for (human) BuChE maybe used.

In this embodiment a sample is provided to a sample inlet after which the sample flows in two capillaries each to a different sensor. The signals obtained from those sensors are processed to calculate the concentrations of uninhibited and total BuChE. This enables calculation of the percentage inhibited BuChE and as such an indication of the exposure to organophosphorous compounds.

In another embodiment the two sensors are placed along one and the same capillary that runs from the sample inlet site. In this embodiment the sample flows across one sensor to measure the concentration of one analyte, and the same sample subsequently flows across the second sensor to measure the other analyte. Of course in this embodiment the probe and/or the antibody may be available as free soluble in the sample solution or bound to the sensor surface. Again, the signals from both sensors are then further processed to ultimately yield the concentration and/or percentage of inhibited BuChE in the sample. Such a sequential arrangement is allowed when the first sensor only binds a small fraction of the analyte (not depeleting the sample) such that the total concentration of analyte in the sample is not markedly affected.

In a further embodiment, the total amount of BuChE is measured by one sensor in one branch of the capillary, while in a second branch of the capillary all uninhibited BuChE is captured by a gel in which an excess amount of probe is available. After capturing all the uninhibited BuChE from the sample, the remaining amount of BuChE, which then necessarily is inhibited BuChE, is measured with immobilized antibodies. In the same embodiment the first measurement in the first capillary may also be a measurement of the amount of uninhibited BuChE, since the total amount of BuChE, and thus the percentage of inhibited BuChE, can be calculated in either way. In a very simplified form of this embodiment, the amount of uninhibited BuChE is measured quantitatively, while still depleting the sample from this uninhibited BuChE. This can be accomplished by having an excess amount of probe on the sensor. Since, beforehand it is not known how high the concentration of uninhibited BuChE will be, it would be possible to accommodate a dilution series of the sample to be measured by a series of sensors, where the sample is split and diluted in such a way that there is at least one sensor which would contain an excess amount of probe in order to completely delete this (diluted) sample.

According to the above description, the analysis device may be a lab-on-a-chip, which has a sample inlet site at which a sample to be assayed, such as a drop of blood, is applied. Optionally, a further aqueous solution containing a free soluble probe or antibody as described above may be added. Further, this 'chip' has one, two or more capillaries in which the sample is further guided to the one, two or more sensors at which the analyte is eventually detected. The signals that are obtained from these, one, two or more sensors are being sent to a calculating unit, which calculating unit may be a micro-computer, which is situated on the chip itself, or the calculating unit may be removed from the sensors and the results are transmitted. Temporarily, the signals can be stored in a storage medium on the chip and can be read out when contacting the chip with a transmission unit or directly with a calculating unit. Contacting the chip with a transmission device or calculating device may be in the form of any kind of contact that is able to transfer the signals, such as cables, a bus-like structure, such as a USB-stick or the like. Alternatively, the signals may be transmitted directly by a wireless transmitter, which may be any kind of wireless transmission system, i.e. modulated or unmodulated, encrypted or non-encrypted, radiosignals, optic, sonic or electromagnetic, etc. Preferably such a wireless transmission system is a WiFi system or an infra-red system.

Preferably, the biosensor system also provides an indicator which generates a signal when an excess amount of inhibited BuChE has been detected. Such an indicator can provide a chemical, electrical, optical or acoustical signal. The signal may be directly produced by one or more of the reagents contained in the system, but it may also be produced indirectly through input from the calculating unit.

As will be appreciated by one skilled in the art, aspects of the present invention may be embodied as a system, method or computer program product. Accordingly, aspects of the present invention may take the form of an entirely hardware embodiment, or an embodiment combining software and hardware aspects that may all generally be referred to herein as a "circuit," "module" or "system." Furthermore, aspects of the present invention may take the form of a computer program product embodied in one or more computer readable medium(s) having computer readable program code embodied thereon. Program code embodied on a computer readable medium may be transmitted using any appropriate medium, including, but not limited to, wireless, wireline, optical fiber cable, RF, etc., or any suitable combination of the foregoing.

### EXAMPLE

An antibody against human butyrylcholinesterase was immobilized on the surface of an integrated optical chip via streptavidin-biotin interactions. Initially, biotin was covalently bound to the chip. Subsequently, streptavidin (40 ug/ml in phosphate buffer saline) was applied and bound to the chip-immobilized biotin and finally a solution with biotinylated antibodies (3E8, 1 ug/ml in phosphate buffer saline) was applied such that the antibody was able to bind to the streptavidin. In order to apply the solutions in a well-controlled manner, a perspex flow cell was adhered to the optical chip with a UV-curing adhesive, such that a well-defined flow channel (Wxh = 3mm x 0.5 mm) above the surface of the optical chips is obtained. After this process the chip is ready to detect binding of human butyrylcholinesterase.

The integrated optical chip carries a ring resonator. The ring resonator has a length of 500 um. During the experiments the shift of the resonances in the optical response (transmission of the ring resonator) was measured. To this end, a tunable laser was continuously swept in steps of 1 pm across 4 nm and for each sweep the transmission of the ring was recorded as function of applied wavelength. From the recorded spectra over time, the shift of the resonance wavelengths as result of refractive index changes caused by human butyrylcholinesterase binding to the antibodies, is determined and plotted as function of time. This experiment was performed for different concentrations of human butyrylcholinesterase in phosphate buffer saline, that were applied to the sensor at a flow rate of 30 ul/min. As is shown in Fig. 1 a dose-dependent shift in resonance wavelength could be observed.

## Claims

1. Optical resonator biosensor for the detection of butyrylcholinesterase in a sample,
comprising two optical resonator biosensor systems in which in the first optical resonator biosensor system a probe is attached to the resonator wherein said probe is a compound that is
able to bind uninhibited butyrylcholinesterase, said probe being able to bind to butyrylcholinesterase at the active site, and in which in the second optical resonator biosensor system an antibody is attached to the resonator, wherein said antibody is able to bind both inhibited and uninhibited butyrylcholinesterase,
wherein either the probe and/or antibody are attached to their respective optical resonator biosensor systems before the biosensors are brought into contact with the sample or
wherein the probe and/or antibody are attached to their respective optical resonator biosensor systems after the moment that the biosensors are brought into contact with the sample.

2. Optical resonator biosensor according to claim 1, wherein a sample is applied in such a way that it comes into contact with both optical resonator biosensor systems.

3. Optical resonator biosensor according to claim 2, wherein the sample is applied via a capillary track.

4. Optical resonator biosensor according to claim 3, wherein both optical resonator biosensor systems are placed along one capillary.

5. Optical resonator biosensor according to claim 4 in which the amount of probes in said first optical resonator biosensor is sufficient large to capture all the uninhibited butyrylcholinesterase.

6. Optical resonator biosensor according to claim 3, wherein one optical resonator biosensor system is placed along one branch of the capillary, while the other optical resonator biosensor system is placed along an other branch of the capillary.

7. Optical resonator biosensor according to any of the previous claims, wherein the resonator is a ring resonator.

8. Optical resonator biosensor according to any of the previous claims, wherein the resonator biosensor is a waveguide.

9. Optical resonator biosensor according to claim 8, wherein said waveguide is part of an interferometer.

10. Method for determining the exposure to an organophosphorous compound by measuring the amounts of inhibited, uninhibited and total butyrylcholinesterase in a sample by assaying said sample with:
- an optical resonator biosensor comprising two optical resonator biosensor systems in which in the first optical resonator biosensor system a probe is attached to the resonator wherein said probe is able to bind uninhibited butyrylcholinesterase, wherein said probe is a compound that is able to bind to buryrylcholinesterase at the active site, and in which in the second optical resonator biosensor system an antibody is attached to the resonator, wherein said antibody is able to bind both inhibited and uninhibited butyrylcholinesterase, and wherein the method comprises introducing said sample to the optical resonator biosensor, or
- an optical resonator biosensor comprising two optical resonator biosensor systems in which a probe that is able to bind to buryrylcholinesterase at the active site, can be attached to the first optical resonator biosensor system and in which and antibody that is able to bind both inhibited and uninhibited butyrylcholinesterase can be attached to the second optical resonator biosensor system, and wherein the method comprises adding the probe and/or antibody to the biosensor at the moment that, or shortly before or after the sample is introduced.

11. Use of an optical resonator biosensor for the determination of the exposure to an organophosphorous compound, wherein said optical resonator biosensor is an optical resonator biosensor according to any of claims 1 - 9.

12. Optical resonator biosensor according to any of claims 1 - 9, a method according to claim 10 or a use according to claim 11, wherein the butyrylcholinesterase is a human butyrylcholinesterase.

13. Optical resonator biosensor according to any of claims 1-9, a method according to claim 10 or a use according to claim 11, wherein the probe comprises the following compound: 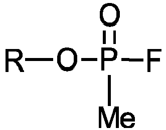 in which R is an spacer chain with more than 10 C-atoms, which is able to be bound to the sensor surface, preferably wherein said spacer chain is a substituted or unsubstituted, linear or branched hydrocarbon chain of more than 10 carbon atoms, wherein one to three of the C atoms may be replaced by a heteroatom selected from the group of O, N and S, and wherein the chain may have substitutions selected from the group of halogen, phenyl, linear or branched O-C₁-C₆ alkyl, linear or branched alkoxy, nitro, cyano or methylsulfinyl.

14. Optical resonator biosensor according to claim 13, a method according to claim 13 or a use according to claim 13, wherein the probe comprises the following compound:

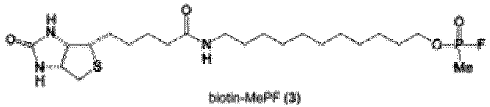

15. Optical biosensor according to any of claims 1-9, 12-14, a method according to claim 10 or a use according to claim 11, wherein the antibody that binds to butyrylcholinesterase is monoclonal antibody 3E8.

## Patentansprüche

1. Optischer Resonator-Biosensor für den Nachweis von Butyrylcholinesterase in einer Probe, umfassend zwei optische Resonator-Biosensorsysteme, bei dem in dem ersten optischen Resonator-Biosensorsystem eine Sonde an den Resonator angebracht ist, wobei die Sonde eine Verbindung ist, die in der Lage ist, nicht inhibierte Butyrylcholinesterase zu binden, wobei die Sonde in der Lage ist, an Butyrylcholinesterase an der aktiven Stelle zu binden, und bei dem in dem zweiten optischen Resonator-Biosensorsystem ein Antikörper an den Resonator angebracht ist, wobei der Antikörper in der Lage ist, sowohl inhibierte als auch nicht inhibierte Butyrylcholinesterase zu binden, wobei beides die Sonde und/oder der Antikörper an ihre jeweiligen optischen Resonator-Biosensorsysteme angebracht werden, bevor die Biosensoren mit der Probe in Kontakt gebracht werden oder
wobei die Sonde und/oder der Antikörper an ihre jeweiligen optischen Resonator-Biosensorsysteme angebracht sind nach dem Moment, in dem die Biosensoren mit der Probe in Kontakt gebracht werden.

2. Optischer Resonator-Biosensor nach Anspruch 1, wobei eine Probe auf so eine Weise appliziert wird, dass sie mit beiden optischen Resonator-Biosensorsystemen in Kontakt kommt.

3. Optischer Resonator-Biosensor nach Anspruch 2, wobei die Probe über eine Kapillarbahn appliziert wird.

4. Optischer Resonator-Biosensor nach Anspruch 3, wobei beide optischen Resonator-Biosensorsysteme entlang einer Kapillare angeordnet sind.

5. Optischer Resonator-Biosensor nach Anspruch 4, bei dem die Menge an Sonden in dem ersten optischen Resonator-Biosensor ausreichend groß ist, um all die nicht-inhibierte Butyrylcholinesterase einzufangen.

6. Optischer Resonator-Biosensor nach Anspruch 3, wobei ein optisches Resonator-Biosensorsystem entlang eines Zweigs der Kapillare angeordnet ist, während das andere optische Resonator-Biosensorsystem entlang eines anderen Zweigs der Kapillare angeordnet ist.

7. Optischer Resonator-Biosensor nach einem der vorhergehenden Ansprüche, wobei der Resonator ein Ringresonator ist.

8. Optischer Resonator-Biosensor nach einem der vorhergehenden Ansprüche, wobei der Resonator-Biosensor ein Wellenleiter ist.

9. Optischer Resonator-Biosensor nach Anspruch 8, wobei der Wellenleiter Teil eines Interferometers ist.

10. Verfahren zum Bestimmen der Exposition gegenüber einer Organophosphorverbindung durch Messen der Mengen an inhibierter, nicht inhibierter und gesamter Butyrylcholinesterase in einer Probe durch Testen der Probe mit:
- einem optischen Resonator-Biosensor, umfassend zwei optische Resonator-Biosensorsysteme, bei dem in dem ersten optischen Resonator-Biosensorsystem eine Sonde an den Resonator angebracht ist, wobei die Sonde in der Lage ist nicht inhibierte Butyrylcholinesterase zu binden, wobei die Sonde eine Verbindung ist, die in der Lage ist an Buryrylcholinesterase an der aktiven Stelle zu binden und bei dem in dem zweiten optischen Resonator-Biosensorsystem ein Antikörper an den Resonator angebracht ist, wobei der Antikörper in der Lage ist, sowohl inhibierte als auch nicht inhibierte Butyrylcholinesterase zu binden, und wobei das Verfahren Einführen der Probe in den optischen Resonator-Biosensor umfasst; oder
- einen optischen Resonator-Biosensor, umfassend zwei optische Resonator-Biosensorsysteme, in dem eine Sonde, die in der Lage ist Buryrylcholinesterase an der aktiven Stelle zu binden, an das erste optische Resonator-Biosensorsystem angebracht werden kann und in dem ein Antikörper, der in der Lage ist, sowohl inhibierte als auch nicht inhibierte Butyrylcholinesterase zu binden, an das zweite optische Resonator-Biosensorsystem angebracht werden kann, und wobei das Verfahren Hinzufügen der Sonde und/oder des Antikörpers zum Biosensor in dem Moment, in dem, oder kurz bevor, oder nachdem die Probe eingeführt wird, umfasst.

11. Verwendung eines optischen Resonator-Biosensors für die Bestimmung der Exposition gegenüber einer Organophosphorverbindung, wobei der optische Resonator-Biosensor ein optischer Resonator-Biosensor nach einem der Ansprüche 1 - 9 ist.

12. Optischer Resonator-Biosensor nach einem der Ansprüche 1 - 9, ein Verfahren nach Anspruch 10 oder eine Verwendung nach Anspruch 11, wobei die Butyrylcholinesterase eine humane Butyrylcholinesterase ist.

13. Optischer Resonator-Biosensor nach einem der Ansprüche 1 - 9, ein Verfahren nach Anspruch 10 oder eine Verwendung nach Anspruch 11, wobei die Sonde die folgende Verbindung umfasst: 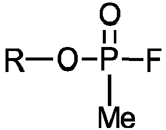 in der R eine Spacerkette mit mehr als 10 C-Atomen ist, die in der Lage ist, an die Sensoroberfläche gebunden zu werden, vorzugsweise wobei die Spacerkette eine substituierte oder unsubstituierte, lineare oder verzweigte Kohlenwasserstoffkette mit mehr als 10 Kohlenstoffatomen ist, wobei ein bis drei der C-Atome durch ein Heteroatom ersetzt werden können, ausgewählt aus der Gruppe von O, N und S, und wobei die Kette Substitutionen haben kann, ausgewählt aus der Gruppe von Halogen, Phenyl, linearem oder verzweigtem O-C₁-C₆-Alkyl, linearem oder verzweigtem Alkoxy, Nitro, Cyano oder Methylsulfinyl.

14. Optischer Resonator-Biosensor nach Anspruch 13, ein Verfahren nach Anspruch 13 oder eine Verwendung nach Anspruch 13, wobei die Sonde die folgende Verbindung umfasst:

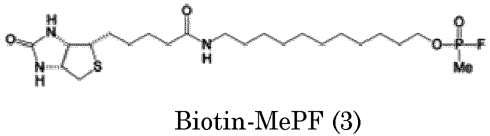

15. Optischer Biosensor nach einem der Ansprüche 1 - 9, 12 - 14, ein Verfahren nach Anspruch 10 oder eine Verwendung nach Anspruch 11, wobei der Antikörper, der an Butyrylcholinesterase bindet, monoklonaler Antikörper 3E8 ist.

## Revendications

1. Biocapteur à résonateur optique pour la détection de butyrylcholinestérase dans un échantillon,
comprenant deux systèmes de biocapteur à résonateur optique dans lesquels, dans le premier système de biocapteur à résonateur, une sonde est fixée au résonateur dans lequel ladite sonde est un composé qui est capable de se lier à une butyrylcholinestérase non inhibée, ladite sonde étant capable se lier à une butyrylcholinestérase au niveau du site actif, et dans lesquels, dans le second système de biocapteur à résonateur optique, un anticorps est fixé au résonateur, ledit anticorps étant capable de se lier à la fois à une butyrylcholinestérase inhibée et non inhibée,
dans lequel la sonde et/ou l'anticorps sont fixés à leurs systèmes respectifs de biocapteur à résonateur optique avant que les biocapteurs ne soient mis en contact avec l'échantillon ou
dans lequel la sonde et/ou l'anticorps sont fixés à leurs systèmes respectifs de biocapteur à résonateur optique après le moment où les biocapteurs sont mis en contact avec l'échantillon.

2. Biocapteur à résonateur optique selon la revendication 1, dans lequel un échantillon est appliqué de telle manière qu'il vient au contact avec les deux systèmes de biocapteur à résonateur optique.

3. Biocapteur à résonateur optique selon la revendication 2, dans lequel l'échantillon est appliqué via une piste capillaire.

4. Biocapteur à résonateur optique selon la revendication 3, dans lequel les deux systèmes de biocapteur à résonateur optique sont placés le long d'un capillaire.

5. Biocapteur à résonateur optique selon la revendication 4, dans lequel la quantité de sondes dans ledit premier biocapteur à résonateur optique est suffisamment importante pour capturer toute la butyrylcholinestérase non inhibée.

6. Biocapteur à résonateur optique selon la revendication 3, dans lequel un système de biocapteur à résonateur optique est placé le long d'une branche du capillaire, alors que l'autre système de biocapteur à résonateur optique est placé le long d'une autre branche du capillaire.

7. Biocapteur à résonateur optique selon l'une quelconque des revendications précédentes, dans lequel le résonateur est un résonateur en anneau.

8. Biocapteur à résonateur optique selon l'une quelconque des revendications précédentes, dans lequel le biocapteur à résonateur est un guide d'onde.

9. Biocapteur à résonateur optique selon la revendication 8, dans lequel ledit guide d'onde est une partie d'un interféromètre.

10. Procédé pour déterminer l'exposition à un composé organophosphoré en mesurant les quantités de butyrylcholinestérase inhibée, non inhibée et totale dans un échantillon en analysant ledit échantillon avec :
- un biocapteur à résonateur optique comprenant deux systèmes de biocapteurs à résonateur optique dans lesquels, dans le premier système de biocapteur à résonateur optique, une sonde est fixée au résonateur dans lequel ladite sonde est capable de se lier à une butyrylcholinestérase non inhibée, dans lequel ladite sonde est un composé qui est capable de se lier à une buryrylcholinestérase au niveau du site actif, et dans lesquels, dans le second système de biocapteur à résonateur optique, un anticorps est fixé au résonateur, dans lequel ledit anticorps est capable de se lier à la fois à une butyrylcholinestérase inhibée et non inhibée, et dans lequel le procédé comprend l'introduction dudit échantillon dans le biocapteur à résonateur optique, ou
- un biocapteur à résonateur optique comprenant deux systèmes de biocapteur à résonateur optique dans lesquels une sonde qui est capable de se lier à une buryrylcholinestérase au niveau du site actif, peut être fixée au premier système de biocapteur à résonateur optique et dans lesquels un anticorps qui est capable de se lier à la fois une butyrylcholinestérase inhibée et non inhibée peut être fixé au second système de biocapteur à résonateur optique, et dans lequel le procédé comprend l'ajout de la sonde et/ou de l'anticorps au biocapteur au moment où l'échantillon est introduit, ou peu avant ou après celui-ci.

11. Utilisation d'un biocapteur à résonateur optique pour la détermination de l'exposition à un composé organophosphoré, dans laquelle ledit biocapteur à résonateur optique est un biocapteur à résonateur optique selon l'une quelconque des revendications 1 à 9.

12. Biocapteur à résonateur optique selon l'une quelconque des revendications 1 à 9, procédé selon la revendication 10 ou utilisation selon la revendication 11, dans lesquels la butyrylcholinestérase est une butyrylcholinestérase humaine.

13. Biocapteur à résonateur optique selon l'une quelconque des revendications 1 à 9, procédé selon la revendication 10 ou utilisation selon la revendication 11, dans lesquels la sonde comprend le composé suivant : 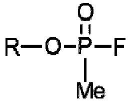 dans lequel R est une chaîne d'espacement ayant plus de 10 atomes de carbone, qui est capable de se lier à la surface de capteur, de préférence dans lequel ladite chaîne d'espacement est une chaîne hydrocarbonée substituée ou non substituée, linéaire ou ramifiée de plus de 10 atomes de carbone, dans lequel un à trois des atomes C peuvent être remplacés par un hétéroatome choisi parmi le groupe de O, N et S, et dans lequel la chaîne peut avoir des substitutions choisies parmi le groupe constitué d'halogène, phényle, O-alkyle en C₁-C₆ linéaire ou ramifié, alcoxy linéaire ou ramifié, nitro, cyano ou méthylsulfinyle.

14. Biocapteur à résonateur optique selon la revendication 13, procédé selon la revendication 13 ou utilisation selon la revendication 13, dans lesquels la sonde comprend le composé suivant :

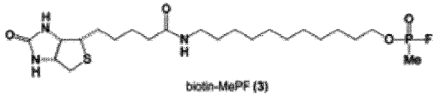

15. Biocapteur optique selon l'une quelconque des revendications 1 à 9, 12 à 14, procédé selon la revendication 10 ou utilisation selon la revendication 11, dans lesquels l'anticorps qui se lie à une butyrylcholinestérase est un anticorps monoclonal 3E8.
